# EUROPEAN PATENT APPLICATION

(11) **EP 2 907 830 A1**
(43) Date of publication of application: **19.08.2015**
(21) Application number: 14155126.7
(22) Date of filing: 14.02.2014
(51) Int. Cl.: C08F 26/06, A61K 8/81, A61K 8/04, C08F 2/14

(54) **Oil-based polymer dispersions for styling applications and cosmetic emulsions**

(71) Applicant: BASF SE, 67056 Ludwigshafen (DE)
(72) Inventor: Graham, David, 40213 Düsseldorf (DE); Burakowska-Meise, Ewelina, 68259 Mannheim (DE); Nguyen-Kim, Son, 69502 Hemsbach (DE); Hössel, Peter, 67105 Schifferstadt (DE); Walter, Hans-Michael, 67251 Freinsheim (DE); Last, Stefan, 67459 Böhl-Iggelheim (DE); Jahnel, Wolfgang, 76756 Bellheim (DE); Wendel, Volker, 64342 Seeheim-Jugenheim (DE); Denuell, Wolfgang, 68163 Mannheim (DE)
(74) Representative: Reinhardt, Jürgen

(57) **Abstract**

The present invention relates to a process for making a copolymer, wherein the copolymer comprises hydrophilic and/or hydrophobic monomers, comprising copolymerization of the monomers in a one-step process in a cosmetically acceptable oil in the presence of common emulsifiers so that a dispersion of the copolymer in the oil is obtained.

## Description

The present invention relates to a process for making a copolymer, wherein the copolymer comprises hydrophilic and/or hydrophobic monomers, comprising copolymerization of the monomers in a one-step process in a cosmetically acceptable oil in the presence of common emulsifiers so that a dispersion of the copolymer in the oil is obtained.

Current precipitation and liquid dispersion polymers show excellent cosmetic properties in a variety of applications such as rheology modification, conditioning, skincare and styling. One of the most significant problems with both types of polymerization process are the limits imposed on which types of monomers can be used, due to initial solubility in the very hydrophobic phase, or in the water phase. The second problem with both processes is the high cost of drying or stripping solvent from the final products.

WO 99/29735 discloses a process for making polyvinylpyrrolidone and for making copolymers of vinylpyrrolidone and other monomers. In the process disclosed the monomers are dissolved in oil, e. g. silicon oil or mineral oil. The polymer that is obtained by polymerization of the monomers precipitates from the oil so that a dispersion of the polymer in oil is formed. The examples of WO 99/29735 disclose lauryl methacrylate and acrylic acid as comonomers that are used together with vinylpyrrolidone.

It has been found an alternative process which offers a solution to the afore mentioned problems which allows a variety of monomers, both hydrophilic and hydrophobic to be copolymerized in a one-step process in the presence of common emulsifiers which requires on expensive drying or solvent stripping procedures.

The solution to this problem was found by polymerizing directly in cosmetically acceptable oils, free from water or other common solvents. The variety and miscibility of cosmetic oils available enables control over the hydrophobicity of the reaction medium and thus allows us to access a broader palette of monomers.

The performance of the polymers was found to have benefits over conventional market standard polymers.

### Example monomers:

Vinyl pyrrolidone from 0,0 - 99,9 weight %
Vinyl Imidazole from 0,1 - 99,9 weight%
Acrylic acid from 0,0 - 80,0 weight%
Methacrylic acid from 0,0 - 80,0 weight %
A C12 - C30 hydrophobic acrylates or methacrylates 0,0 - 25%
Surface active acrylate or methacrylate with a PEG building block from 5-50 units and a hydrophobic tail of C8 - C25 0,0 -30%
A monomer capable of forming hydrogen bonds such as ureido-methacrylate 0,0 - 30%
Acrylic or methacrylic acid esters C1 - C6 0,0 - 30%
Cross linkers such as (but not limited to) Pentaerythritol triallyl ether, and derivatives of glycerol triacrylates 0,0 - 5,0%

### Example cosmetic oils:

Parafinum liquidum, Myritol^{®} types, migliol^{®} types, oils based on caprylic/capric triglycerides, and their mixtures with parafinum liquidium, and mygliol^{®} types.

### Example emulsifiers:

Emulgade^{®}, Span^{®}, Tween^{®} and Cremophor^{®} types.

A further subject of the present invention is the copolymer obtainable by the process according to any of claims 1 to 5.

A further subject of the present invention is the dispersion obtainable by the process according to any of claims 1 to 5.

## Claims

1. A process for making a copolymer, wherein the copolymer comprises hydrophilic and/or hydrophobic monomers, comprising copolymerization of the monomers in a one-step process in a cosmetically acceptable oil in the presence of common emulsifiers so that a dispersion of the copolymer in the oil is obtained.

2. The process according to claim 1, wherein the oil is free from water or other common solvents.

3. The process according to claim 1 or 2, wherein the monomers used are
0.0 - 99.9 weight % Vinyl pyrrolidone
0.1 - 99.9 weight% Vinyl Imidazole
0.0 - 80.0 weight% Acrylic acid
0.0 - 80.0 weight % Methacrylic acid
0.0 - 25 weight% C12 - C30 hydrophobic acrylates or methacrylates
0.0 - 30 weight % Surface active acrylate or methacrylate with a PEG building block from 5-50 units and a hydrophobic tail of C8 -C25
0.0 - 30 weight% monomer capable of forming hydrogen bonds such as ureido-methacrylate Acrylic or methacrylic acid esters C1 - C6
0.0 - 5.0 weight% Cross linkers such as Pentaerythritol triallyl ether, and derivatives of glycerol triacrylates.

4. The process according to any of claims 1 to 3, wherein the oil is selected from the group consisting of
Parafinum liquidum, Myritol^{®} types, migliol^{®} types, oils based on caprylic/capric triglycerides, and their mixtures with parafinum liquidum, and mygliol^{®} types.

5. The process according to any of claims 1 to 4, wherein the emulsifier is selected from the group consisting of
Emulgade^{®}, Span^{®}, Tween^{®} and Cremophor^{®} types.
